(19) Europäisches Patentamt
European Patent Office
Office européen des brevets

(11) Veröffentlichungsnummer: **0 262 388 B1**

(12) **EUROPÄISCHE PATENTSCHRIFT**

(45) Veröffentlichungstag der Patentschrift: **04.03.92**

(21) Anmeldenummer: **87112319.6**

(22) Anmeldetag: **25.08.87**

(51) Int. Cl.5: **C07C 69/06**, C07C 69/14, C07C 69/24, C07C 43/184, C07C 67/08, C07C 41/20, C07C 29/20, A61K 7/46

# (54) 2-tert.-Butyl-4-methyl-cyclohexanol-derivate, deren Herstellung und Verwendung als Riechstoffe.

(30) Priorität: **30.08.86 DE 3629603**

(43) Veröffentlichungstag der Anmeldung: **06.04.88 Patentblatt 88/14**

(45) Bekanntmachung des Hinweises auf die Patenterteilung: **04.03.92 Patentblatt 92/10**

(84) Benannte Vertragsstaaten: **CH DE ES FR GB LI NL**

(56) Entgegenhaltungen:
**EP-A- 0 002 510**
**FR-A- 2 577 922**

(73) Patentinhaber: **BASF Aktiengesellschaft**
**Carl-Bosch-Strasse 38**
**W-6700 Ludwigshafen(DE)**

(72) Erfinder: **Gramlich, Walter, Dr.**
**Auf der Hoehe 11**
**W-6803 Edingen-Neckarhausen(DE)**
Erfinder: **Hoffmann, Werner, Dr.**
**14 Horatio Street, Apt. 11D**
**New York, N.Y. 10014(US)**
Erfinder: **Schuster, Ludwig, Dr.**
**Weinheimer Strasse 44**
**W-6703 Limburgerhof(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft 2-tert.-Butyl-4-methyl-cyclohexanolderivate der allgemeinen Formel I

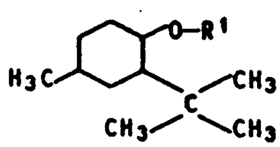

(I),

in der $R^1$ für eine Alkylgruppe mit 1 bis 3 C-Atomen oder eine Acylgruppe

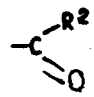

steht, in der $R^2$ ein Wasserstoffatom oder einen Alkylrest mit 1 bis 5 C-Atomen bedeutet. Acylgruppen, insbesondere die Acetylgruppe werden bevorzugt.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmittel, Kosmetika, Leime etc. hat die Riechstoffindustrie ständig Bedarf an neuen Riechstoffen, die allein oder in Form von Kompositionen wertvolle Parfüms bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es war daher die Aufgabe der Erfindung, neue interessante Riechstoffe zu entwickeln, die auf möglichst einfache Weise aus gut zugänglichen und daher billigen und kommerziell erhältlichen Ausgangsstoffen hergestellt werden können.

In den erfindungsgemäßen 2-tert.-Butyl-4-methyl-cyclohexanolderivaten der Formel I wurden Verbindungen mit z.T. außerordentlich interessanten Riechstoffeigenschaften gefunden, die auf einfache Weise aus gut zugänglichen Ausgangsstoffen hergestellt werden können.

Ausgangsstoff für die Herstellung der erfindungsgemäßen Verbindungen ist das 2-tert.-Butyl-4-methyl-phenol, welches leicht aus p-Kresol durch tert.-Butylierung mit Isobutylen hergestellt werden kann und darüberhinaus kommerziell verfügbar ist. Dieses Dialkylphenol wird entweder zuerst acyliert bzw. alkyliert und dann einer Kernhydrierung unterworfen oder aber zunächst zum 2-tert.-Butyl-4-methyl-cyclohexanol hydriert und dann acyliert bzw. alkyliert.

Gegenstand der Erfindung ist daher auch ein Verfahren zur Herstellung von 2-tert.-Butyl-4-methyl-cyclohexanolderivaten der allgemeinen Formel I, das dadurch gekennzeichnet ist, daß man ein 2-tert.-Butyl-4-methyl-phenolderivat der allgemeinen Formel II

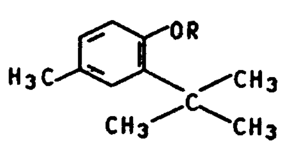

(II),

in der R für Wasserstoff steht oder die oben angegebene Bedeutung hat, hydriert und entweder davor oder danach verestert bzw. verethert.

Einige andere alkylierte Cyclohexanolderivate finden bereits Verwendung als Riech- und Aromastoffe. So ist es z.B. 2-tert.-Butyl-cyclohexylacetat, ein im Handel erhältlicher Riechstoff, der einen Kiefer-artigen, holzig-fruchtigen Duft aufweist mit einer zusätzlichen Grün-Note.

2-Isobutyl-cyclohexylacetat weist eine starke Frucht-Note auf, die etwas an Himbeeren erinnert sowie auch einen fruchtigen Geschmack.

4-tert.-Butyl-cyclohexylacetat, ist ein von verschiedenen Firmen vertriebener Riechstoff, der einen süßen, fast 'cremig'-holzigen Duft mit einer weichen blumigen Note aufweist.

Das reine cis-4-tert.-Butyl-cyclohexylacetat, das von IFF unter dem Namen Vertenex® HC vertrieben

wird, weist eine reine Frucht-Note auf.

Ortho/para-tert.-Butyl-cyclohexylcaproate weist eine fruchtig-süße Note mit einem Benzaldehyd-artigen Unterton auf.

Die erfindungsgemäßen Verbindungen der Formel I weisen jedoch wesentlich andere, recht unterschiedliche, aber äußerst interessante Duftnoten auf.

So besitzt beispielsweise das 2-tert.-Butyl-4-methyl-cyclohexylacetat einen hochinteressanten animalisch-holzigen Duft mit einer trockenen Tabak-Note, der zusätzlich an natürlichen Moschus und Civet erinnert. Das 2-tert.-Butyl-4-methyl-cyclohexylpropionat weist eine interessante fruchtige Note auf, die an Aprikosen und Pflaumen erinnert.

Die Kernhydrierung von 2-tert.-Butyl-4-methyl-phenol bzw. dessen Estern und Alkylethern erfolgt auf an sich bekannte Weise durch katalytische Hydrierung in Gegenwart von bekannten Hydrierkatalysatoren, wie Raney-Nickel, Palladium-, Rhodium- oder Ruthenium-Katalysatoren, vorzugsweise an Rutheniumkatalysatoren.

Je nach den angewandten Reaktionsbedingungen erhält man unterschiedliche Diastereomerengemische, die jedoch alle olfaktorisch interessante Eigenschaften aufweisen.

Hydriert man beispielsweise bei einem Wasserstoffdruck von 50 bar und einer Temperatur von 120 °C, so erhält man ein Isomerenverhältnis von A : B : C : D = 68 : 29 : 2 : 1 der im folgenden Schema dargestellten Isomeren. Hydriert man bei 50 bar Wasserstoffdruck und einer Temperatur von 200 °C, so beträgt das Verhältnis A : B : C : D = 52 : 24 : 16 : 8. Die Isomerenverhältnisse wurden jeweils NMR-spektroskopisch bestimmt ($^{1}$H- und $^{13}$-C-NMR-Spektren).

**Schema**

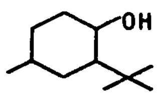
A

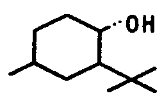
B

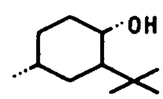
C

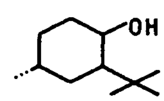
D

Durch Methylierung des Zwischenproduktes 2-tert.-Butyl-4-methyl-cyclohexanol mit Alkylhalogeniden oder Dialkylsulfaten erhält man die entsprechenden Alkylether der Formel I.

Diese lassen sich in noch besseren Ausbeuten dadurch erhalten, daß man das 2-tert.-Butyl-4-methylphenol zunächst alkyliert und dann erst kernhydriert. Diese O-Alkylierung läßt sich nach vielen in der Literatur beschriebenen Methoden (vgl. Houben-Weyl, Methoden der organischen Chemie, Band VI/3, Seiten 54 ff. [1965]) durchführen, beispielsweise mit Dialkylsulfaten, Alkylhalogeniden, Arylsulfonsäureestern oder alkylschwefelsauren Salzen. Anschließend wird dann der substituierte Phenolether wie oben beschrieben hydriert.

Die 2-tert.-Butyl-4-methyl-cyclohexyl-alkylether weisen interessante erdig-holzige Vetiver-artige Geruchsnoten auf.

Zu olfaktorisch noch interessanteren Riechstoffen gelangt man durch Veresterung des 2-tert.-Butyl-4-methyl-cyclohexanols. Hierzu kann man Carbonsäureanhydride, wie Acetanhydrid, meist in Gegenwart von katalytischen Mengen Phosphorsäure oder in Gegenwart eines anderen üblichen Katalysators, wie Alkalisalzen der entsprechenden Carbonsäure einsetzen. Ebensogut läßt sich die Veresterung der Cyclohexanole unter Verwendung von Acylhalogeniden wie Acetylchlorid durchführen. Hierbei arbeitet man zweckmäßigerweise in Anwesenheit von tertiären Aminen, wie Pyridin oder Dimethylanilin bzw. in Anwesenheit von Alkali- oder Erdalkaliacetaten oder anderer organischer Basen.

Die erhaltenen Ester werden zweckmäßig durch Destillation unter verminderten Druck gereinigt; sie stellen farblose bis leicht gelblich gefärbte Flüssigkeiten dar, sind unlöslich in Wasser aber löslich in organischen Lösungsmitteln, wie Alkoholen, Ethern, Ketonen, Estern und Kohlenwasserstoffen.

Der interessanteste der neuen Ester ist das 2-tert.-Butyl-4-methyl-cyclohexylacetat, welches beispielsweise aus dem 2-tert.-Butyl-4-methyl-cyclohexanol-Diastereomerengemisch durch Acetylierung mit Acetanhydrid erhältlich ist und einen interessanten animalisch-holzigen Duft mit einer trockenen Tabak-Note aufweist und zusätzlich an natürlichen Moschus und Civet erinnert.

Die erwähnten Cyclohexanol-Ester lassen sich daneben durch Acetylierung von 2-tert.-Butyl-4-methylphenol und anschließende Kernhydrierung herstellen, jedoch sind die Ausbeuten hierbei erheblich schlechter.

Aufgrund der beschriebenen Geruchseigenschaften können die neuen Verbindungen der allgemeinen

Formel I vorteilhaft als Riechstoffe oder Bestandteile von Riechstoffkompositionen und Parfümölen für kosmetische und technische Anwendungen eingesetzt werden.

Besonders interessante Verbindungen sind:
2-tert.-Butyl-4-methyl-cyclohexyl-formiat,
2-tert.-Butyl-4-methyl-cyclohexyl-acetat,
2-tert.-Butyl-4-methyl-cyclohexyl-propionat,
2-tert.-Butyl-4-methyl-1-methoxy-cyclohexan.

In den folgenden Beispielen wird die Herstellung einiger der neuen Verbindungen der allgemeinen Formel I exemplarisch beschrieben sowie ein anwendungstechnisches Beispiel angeführt.

Beispiel 1

a) Herstellung von 2-tert.-Butyl-4-methyl-cyclohexanol

In einem Autoklaven wurde eine Mischung aus 400 g (2.44 Mol) 2-tert.-Butyl-4-methyl-phenol. 400 ml Dioxan sowie 1 g Rutheniumhydroxid vorgelegt und bei einer Temperatur von 120 °C und einem Wasserstoffdruck von 50 bar bis zur Druckkonstanz hydriert (Gesamthydrierzeit ca. 3 1/2 Stunden). Nach Abtrennen des Katalysators wurde das Dioxan abdestilliert und der Rückstand bei 0.01 mbar fraktioniert. Nach einer kleinen VorlaufFraktion (15 g vom Kp. bis 60 °C/0,01 mbar) destillierten bei einer Temperatur von 80 bis 85 °C/0.01 mbar 401 g (2.36 Mol, entsprechend einer Ausbeute von 96 %) 2-tert.-Butyl-4-methyl-cyclohexanol über, die nach dem Abkühlen zu einer halbfesten Masse erstarrten. Nach $^{13}$C-NMR-spektroskopischen Daten konnten sie als ein Diastereomerengemisch folgender Zusammensetzung identifiziert werden: A : B : C : D = 68 : 29 : 2 : 1

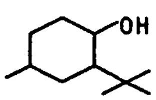
A

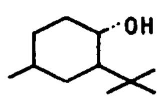
B

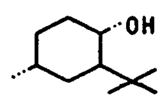
C

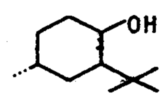
D

Je nach angewendeter Reaktionstemperatur und Wasserstoffdrücken gelangte man zu unterschiedlichen Diastereomerenverhältnissen an A, B, C und D, wie in der folgenden Tabelle veranschaulicht ist.

Tabelle

| Beispiel | Temperatur [ °C] | Wasserstoffdruck [bar] | Diastereomeren-Verhältnis | | | |
|---|---|---|---|---|---|---|
| | | | A | B | C | D |
| 1a | 120 | 50 | 68 | 29 | 2 | 1 |
| 1b | 160 | 50 | 61 | 28 | 9 | 2 |
| 1c | 180 | 50 | 58 | 23 | 14 | 5 |
| 1d | 200 | 50 | 53 | 24 | 16 | 7 |

b) Herstellung von 2-tert.-Butyl-4-methyl-cyclohexyl-formiat

Ein Gemisch aus 920 g (20 Mol) Ameisensäure und 46 g Wasser wurde in einem Kolben vorgelegt und hierzu bei 25 °C innerhalb von 30 Minuten 170 g (1 Mol) des gemäß 1a) erhaltenen 2-tert.-Butyl-4-methyl-cyclohexanol (Diastereomerengemisch) zugetropft. Nach 2-tägigem Rühren bei Raumtemperatur (Rt) wurde die unumgesetzte Ameisensäure unter vermindertem Druck (200 mbar) abdestilliert und der Rückstand über Calciumoxid bei 0,03 mbar fraktioniert. Man erhielt 190 g (entsprechend 96 % Ausbeute) eines Diastereomerengemischs von drei Formiaten.
Das Gemisch weist eine interessante Holznote auf.

Beispiel 2

2-tert.-Butyl-4-methyl-cyclohexyl-acetat

Ein Gemisch aus 680 g (4 Mol) 2-tert.-Butyl-4-methyl-cyclohexanol und 612 g (6 Mol) Acetanhydrid wurde auf eine Temperatur von 145-160 °C erhitzt und dabei die gebildete Essigsäure innerhalb von 3 h langsam abdestilliert. Nach Reaktionsende wurde abgekühlt, das überschüssige Acetanhydrid bei 20 mbar abdestilliert, der Rückstand nach dem Abkühlen in 500 ml Diethylether aufgenommen, die etherische Lösung mit wäßriger Natriumhydrogencarbonat-Lösung Säure-frei gewaschen und nach Entfernen des Lösungsmittels der Rückstand fraktioniert. Nach einer kleinen Vorlauf-Fraktion (13 g vom Kp. = 30-45 °C/0,005 mbar) destillierten bei einer Temperatur von 54-56 °C/0,005 mbar 806 g 2-tert.-Butyl-4-methyl-cyclohexylacetat über. Brechungsindex $n_D^{25}$ = 1.4505. Die Ausbeute betrug 95 % der Theorie.

Die Verbindung stellt ein Diastereomerengemisch gleicher Zusammensetzung wie der Ausgangsverbindung dar und weist einen hochinteressanten animalisch-holzigen Duft mit einer trockenen Tabak-Note auf, die zusätzlich an natürlichen Moschus und Civet erinnert.

Beispiel 3

2-tert.-Butyl-4-methyl-cyclohexylpropionat

Eine Mischung aus 340 g (2 Mol) 2-tert.-Butyl-4-methyl-cyclohexanol und 650 g (5 Mol) Propionsäureanhydrid wurde analog Beispiel 2 auf 162-185 °C Innentemperatur erhitzt, wobei gleichzeitig innerhalb von ca. 3 h die gebildete Propionsäure abdestilliert wurde. Nach analoger Aufarbeitung erhielt man bei der Destillation unter vermindertem Druck 411 g (90 % Ausbeute) 2-tert.-Butyl-4-methyl-cyclohexylpropionat vom Siedepunkt Kp = 37 °C/0,001 mbar und Brechungsindex $n_D^{25}$ = 1.4512. Auch das Propionat stellt ein Diastereomerengemisch dar; es weist eine interessante fruchtige Note auf, die an Aprikosen und Pflaumen erinnert.

Beispiel 4

a) 2-tert.-Butyl-4-methyl-1-phenylmethylether

Zu einer Lösung aus 339 g (2,07 Mol) 2-tert.-Butyl-4-methyl-phenol in 1 l Toluol tropfte man zunächst 304 g (200 ml, 3,8 Mol) einer 50 gew.%igen wäßrigen Natronlauge und dann im Verlauf einer Stunde unter Kühlung auf 20 bis 40 °C 315 g (237 ml, 2,5 Mol) Dimethylsulfat. Dann wurde das Reaktionsgemisch 12 h bei 25 °C nachgerührt. Anschließend verdünnte man mit 1 l Wasser sowie 250 ml einer 20 gew.%igen wäßrigen Natronlauge, rührte 1 h bei 25 °C, trennte die organische Phase ab, wusch letztere mit 100 ml Wasser und entfernte danach das Toluol. Der Rückstand wurde fraktioniert destilliert. Man erhielt 300 g 2-tert.-Butyl-4-methyl-1-phenyl-methylether vom Kp. = 65 °C/2 mbar.

Die Verbindung weist eine unangenehme Chinolin-Note auf.

b) 2-tert.-Butyl-4-methyl-cyclohexyl-methylether

300 g des gemäß a) hergestellten Phenolethers wurden analog Beispiel 1 in Gegenwart von Rutheniumhydroxid hydriert. Man erhielt nach Entfernen des Lösungsmittels Dioxan ein leicht gelbliches Öl, das bei 0,15 mbar fraktioniert werden konnte.
Die Verbindung weist eine milde würzig-kampfrige Note auf.

| Anwendungsbeispiel 1 | | |
|---|---|---|
| Chypre-Komposition | A ohne I-Zusatz [Gew.-Teile] | B mit I-Zusatz [Gew.-Teile] |
| Cyclododecyl-tert.-butylether (BASF) | 120 | 120 |
| Phenylethyl-phenylacetat | 100 | 100 |
| Bergamottöl | 100 | 100 |
| α-Ionon | 100 | 100 |
| α-Hexylzimtaldehyd | 100 | 100 |
| Benzylacetat | 50 | 50 |
| Vetivenylacetat | 80 | 80 |
| Citronellol | 70 | 70 |
| Linalool | 70 | 70 |
| Cyclopentenylpropionat (BASF) | 50 | 50 |
| Patchouliöl | 30 | 30 |
| Sandelholzöl | 30 | 30 |
| Eichenmoos absolut | 30 | 30 |
| Eugenol | 30 | 30 |
| Labdanumöl französisch | 5 | 5 |
| Corianderöl | 5 | 5 |
| Diethylphthalat | 30 | - |
| 2-tert.-Butyl-4-methyl-cyclohexylacetat | - | 30 |
| | 1000 | 1000 |

Die oben beschriebene typische Chypre-Komposition (A) wirkt durch Zusatz von 30 Teilen 2-tert.-Butyl-4-methyl-cyclohexylacetat (B) anstelle von Diethylphthalat angenehm abgerundet, der Duft zeichnet sich durch mehr Charakter und Fülle aus.

| Anwendungsbeispiel 2 | | |
|---|---|---|
| Seifenparfüm | A ohne I-Zusatz [Gew.-Teile] | B mit I-Zusatz [Gew.-Teile] |
| Coumarin | 20 | 20 |
| p-tert.-Butyl-cyclohexylacetat | 140 | 140 |
| Aldehyd $C_{11}$ (Undecylen) 10 %ig in DPG | 30 | 30 |
| Linalool | 100 | 100 |
| Galaxolid® 50 (JFF) | 50 | 50 |
| Dimethyltetrahydrobenzaldehyd 10 %ig in DPG | 5 | 5 |
| Dihydromyrcenol | 30 | 30 |
| Dimethylheptanol | 15 | 15 |
| Lysmeral* (BASF) | 150 | 150 |
| Isoamylsalicylat | 30 | 30 |
| Cedrylacetat | 70 | 70 |
| Linalylacetat | 70 | 70 |
| Phenylethylmethylether | 20 | 20 |
| Methyljonon gamma | 70 | 70 |
| Phenylethylalkohol | 70 | 70 |
| Citronellol | 40 | 40 |
| Aldehyd $C_{12}$ MNA 10 %ig in DPG | 20 | 20 |
| Vetiverylacetat | 30 | 30 |
| Anisaldehyd | 10 | 10 |
| 2-tert.-Butyl-4-methyl-cyclohexylacetat | - | 30 |
| Dipropylenglykol (DPG) | 30 | - |

* angemeldetes Warenzeichen

Das oben beschriebene Seifenparfüm (A) erhält bereits durch Zusatz von nur 3 Gew.% an dem 2-tert.-Butyl-4-methyl-cyclohexylacetat anstelle von der gleichen Menge Dipropylenglykol eine erheblich frischere

Note mit sehr viel mehr Ausstrahlung.

## Patentansprüche

1. 2-tert.-Butyl-4-methyl-cyclohexanolderivate der allgemeinen Formel I

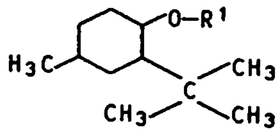

(I),

in der $R^1$ für eine Alkylgruppe mit 1 bis 3 C-Atomen oder eine Acylgruppe

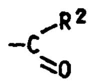

steht, in der $R^2$ ein Wasserstoffatom oder einen Alkylrest mit 1 bis 5 C-Atomen bedeutet.

2. 2-tert.-Butyl-4-methyl-cyclohexyl-formiat.

3. 2-tert.-Butyl-4-methyl-cyclohexyl-acetat.

4. 2-tert.-Butyl-4-methyl-cyclohexyl-propionat.

5. 2-tert.-Butyl-4-methyl-1-methoxy-cyclohexan.

6. Riechstoffkomposition, gekennzeichnet durch einen Gehalt an 2-tert.-Butyl-4-methyl-cyclohexanolderivaten der Formel I gemäß Anspruch 1.

7. Verwendung von 2-tert.-Butyl-4-methyl-cyclohexanolderivaten der Formel I gemäß Anspruch 1 als Riechstoffe.

8. Verfahren zur Herstellung von 2-tert.-Butyl-4-methyl-cyclohexanolderivaten der allgemeinen Formel I, dadurch gekennzeichnet, daß man ein 2-tert.-Butyl-4-methyl-phenolderivat der allgemeinen Formel II

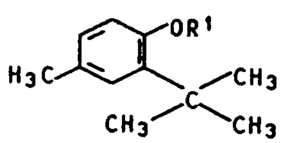

(II),

in der $R^1$ für Wasserstoff steht oder die in Anspruch 1 angegebene Bedeutung hat, hydriert und entweder davor oder danach verestert bzw. verethert.

## Claims

1. A 2-tert-butyl-4-methylcyclohexanol derivative of the formula I

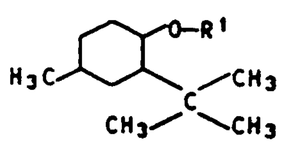

(I)

where $R^1$ is alkyl of 1 to 3 carbon atoms or an acyl group

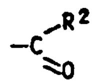

where $R^2$ is hydrogen or alkyl of 1 to 5 carbon atoms.

**2.** 2-tert-Butyl-4-methylcyclohexyl formate.

**3.** 2-tert-Butyl-4-methylcyclohexyl acetate.

**4.** 2-tert-Butyl-4-methylcyclohexyl propionate.

**5.** 2-tert-Butyl-4-methyl-l-methoxycyclohexane.

**6.** A scent composition which contains a 2-tert-butyl-4-methylcyclohexanol derivative of the formula I as claimed in claim 1.

**7.** The use of a 2-tert-butyl-4-methylcyclohexanol derivative of the formula I as claimed in claim 1 as a scent.

**8.** A process for preparing 2-tert-butyl-4-methylcyclohexanol derivatives of the formula I, which comprises hydrogenating a 2-tert-butyl-4-methylphenol derivative of the formula II

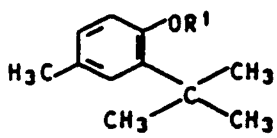

(II),

where $R^1$ is hydrogen or is as defined in claim 1, with prior or subsequent esterification or etherification.

**Revendications**

**1.** Dérivés de 2-tert.-butyl-4-méthyl-cyclohexanol de formule générale I

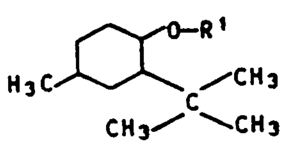

(I),

dans laquelle $R^1$ est mis pour un groupement alkyle ayant de 1 a 3 atomes de carbone au un groupement acyle

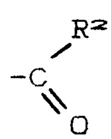

où $R^2$ est un atome d'hydrogène ou un reste alkyle ayant de 1 à 5 atomes de carbone.

**2.** Formiate de 2-tert.-butyl-4-méthyl-cyclohexyle.

**3.** Acétate de 2-tert.-butyl-4-méthyl-cyclohexyle.

**4.** Propionate de 2-tert.-butyl-4-méthyl-cyclohexyle.

**5.** 2-tert.-Butyl-4-méthyl-1-méthoxy-cyclohexane.

**6.** Composition parfumée, caractérisée en ce qu'elle contient un dérivé de 2-tert.-butyl-4-méthyl-cyclohexanol de formule I selon la revendication 1.

**7.** Utilisation de dérivés de 2-tert.-butyl-4-methyl-cyclohexanol de formule I selon la revendication 1 comme parfums.

**8.** Procédé de preparation de dérivés de 2-tert.-butyl-4-méthyl-cyclohexanol de formule générale I, caractérisé en ce qu'on hydrogène un dérivé de 2-tert.-butyl-4-méthyl-phénol de formule générale II

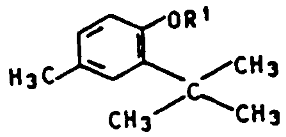

(II).

dans laquelle $R^1$ est mis pour un atome d'hydrogène ou a la signification donnée dans la revendication 1 et on l'éthérifie ou on l'estérifie soit avant soit après.